(19) **Europäisches Patentamt / European Patent Office / Office européen des brevets**

(11) **EP 3 936 625 A1**

(12) **EUROPEAN PATENT APPLICATION**

(43) Date of publication:
**12.01.2022 Bulletin 2022/02**

(51) Int Cl.:
*C12Q 1/6883* (2018.01)     *G01N 33/50* (2006.01)

(21) Application number: **20382620.1**

(22) Date of filing: **09.07.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Fundació Institut d'Investigació Biomèdica de Bellvitge (IDIBELL)**
  **08908 L'Hospitalet de Llobregat (ES)**
• **THE REGENTS OF THE UNIVERSITY OF CALIFORNIA**
  **Oakland, CA 94607-5200 (US)**

(72) Inventors:
• **BESTARD MATAMOROS, Oriol**
  **08908 L'HOSPITALET DE LLOBREGAT (ES)**
• **SARWAL, Minnie**
  **PORTOLA VALLEY, CA California 94028 (US)**
• **PINEDA SAN JUAN, Silvia**
  **SAN FRANCISCO, ca California 94143 (US)**
• **SUR, Swastika**
  **SAN FRANCISCO, ca California 94143 (US)**
• **SIROTA, Marina**
  **SAN FRANCISCO, ca California 94143 (US)**

(74) Representative: **ZBM Patents - Zea, Barlocci & Markvardsen**
  **Rambla Catalunya, 123**
  **08008 Barcelona (ES)**

(54) **DIAGNOSIS OF ALLOGRAFT REJECTION**

(57)     The application refers to biomarkers for diagnosing allograft rejection, in particular, when the underlying cause is antibody-mediated rejection. The application also refers to an *in vitro* method for diagnosing allograft rejection in a patient, the method comprising determining in an isolated sample from the patient the level of expression of *SIGLEC17P* and/or of at least one of its associated downstream coding genes selected from the group consisting of *AP4S1, ZMYM6, USP21, DMAP1, SUPT5H, TP53BP1, NECAB3, BTD, DACT1,* and combinations thereof, wherein when the level of expression of *SIGLEC17P* and/or of at least one of its associated downstream coding genes is higher than a reference value, this is indicative of allograft rejection.

EP 3 936 625 A1

**Description**

**Technical Field**

The present invention is related to the field of allograft transplantation and, in particular, to the diagnosis of allograft rejection.

**Background Art**

To date, organ transplantation remains the preferred treatment for end stage organ disease. However, chronic immune-mediated allograft rejection, fundamentally driven by the humoral effector pathway of adaptive immunity, remains the main cause of accelerated graft loss. Since effective treatment for rejection episodes is available, early diagnosis of this potentially reversible graft injury is imperative. The gold standard technique for the diagnosis of transplant rejection is still a tissue allograft biopsy. However, allograft biopsies cannot be performed systematically and be generalized in clinical practice, as they are costly, invasive and are not capable of predicting subsequent allograft damage. Therefore, there is a need for novel diagnosis methods that are secure and noninvasive.

Moreover, clinical and experimental evidence suggest that chronic antibody-mediated rejection (AMR) progressively appears as a continuum process, preceded by T-cell immune activation, which can lead to clinical or subclinical T-cell mediated rejection (TCMR). AMR was first described for kidney transplants as a distinct clinicopathological entity in 1997 but has later been shown to cause failure of other organ transplantations such as heart, lung, pancreas and, more recently, liver transplantations. In the era of calcineurin inhibitors, AMR (rather than the cell mediated rejection processes) has become a pathologically and epidemiologically important phenotype. The incidence of subclinical AMR, which results in a low 8-year graft survival rate, is on the rise.

AMR and TCMR are, therefore, the two common types of allograft rejection and each have a distinct pathological phenotype/ mechanism. Importantly, treatment of AMR and TCMR can be quite different and require specific therapies. While the widely applied calcineurin inhibitors are helpful in controlling TCMR, they do not affect AMR, which may, in the absence of a convenient therapeutic intervention, proceed to damage the transplanted tissue.

Despite the relevance in clinical management and graft survival, no reliable methods exist nowadays that may effectively differentiate between AMR and TCMR to enable optimizing the treatment for allograft rejection.

Therefore, there is an unmet need to provide non-invasive methods for the early diagnosis of renal allograft rejection and, moreover, for the differential diagnosis of AMR and TCMR.

**Summary of Invention**

The invention disclosed herein provides for a non-invasive and rapid method of detecting allograft tissue rejection in a patient (e.g., human) who has received a transplant. The inventors have found that the blood of patients that are suffering from allograft rejection have significant and consistently increased levels of expression of Sialic Acid Binding Ig Like Lectin 17 Pseudogene *(SIGLEC17P)* as compared to patients with stable (non-damaged) allograft.

A first aspect of the invention therefore refers to an *in vitro* method for diagnosing allograft rejection in a patient, the method comprising determining in an isolated sample from the patient the level of expression of Sialic Acid Binding Ig Like Lectin 17 Pseudogene (*SIGLEC17P*) wherein when the level of expression of *SIGLEC17P* is higher than a reference value, this is indicative of allograft rejection.

The method of the invention does not require providing a tissue allograft biopsy. Instead, the diagnosis according to the invention may be performed by simply providing a blood sample. This method is therefore not invasive.

The inventors have further observed that the upregulation of *SIGLEC17P* is directly related to the physiopathological mechanisms of the specific type allograft rejection. Consequently, the present method provides for fast and non-invasive diagnosis of patients undergoing allograft rejection, without needing an invasive procedure. The fast diagnosis allows those patients to be treated sooner, thereby increasing allograft survival rates.

Importantly, as shown in the examples below, the inventors have found that the *SIGLEC17P* pseudogene is over-expressed among patients whose rejection is antibody-mediated rejection (AMR), but not in patients undergoing T cell-mediated rejection (TCMR). The method of the invention has therefore the very important advantage of determining the underlying mechanism of the rejection, i.e. for distinguishing AMR from TCMR. Prior studies have provided methods for the non-invasive diagnosis of allograft rejection also in peripheral blood, either in for cause biopsies such as the so called kSORT Assay (PLoS Med. 2014 Nov 11;11(11):e1001759) or in surveillance biopsies (Friedewald, J. et al. doi.org/10.1111/ajt.15011), but said methods cannot differentiate between AMR and TCMR. In contrast, the present invention features a method for the differential diagnosis of AMR from TCMR, which is rapid, non-invasive and allows for early detection of the ongoing rejection process. Not only is it the first time that AMR may be differentially diagnosed but, additionally, that can be diagnosed in blood samples. This is of outmost importance as these two types of allograft rejections are driven by two totally distinct effector immune mechanisms and thus, they need different immunosuppressive rescue therapies.

Interestingly, the inventors have further found that 9 *SIGLEC17P* direct downstream coding genes, namely, *AP4S1, ZMYM6, USP21, DMAP1, SUPT5H, TP53BP1, NECAB3, BTD* and *DACT1,* are also overexpressed in patients developing AMR but not in stable transplant recipients with pristine histology and patients undergoing TCMR. *SIGLEC17P* and its associated downstream coding genes are therefore a molecular signature that provides an accurate diagnosis of AMR. The fact that these associated coding genes are also overexpressed exclusively in AMR further confirms the diagnostic capability of the method. Besides, determining the level of expression of one or more of the associated coding genes in addition to that of *SIGLEC17P* in the method of the invention further increases the robustness of the diagnosis.

As mentioned above, differentiating between AMR and TCMR is clinically relevant, since they require different rescue immunosuppressive therapies. Having the ability to distinguish different types of rejection in a non-invasive manner, it can help clinicians to determine appropriate treatments for patients undergoing allograft rejection. This peripheral molecular signature may not only allow for a fast and non-invasive detection of an on-going AMR, but also i) indicate an on-going AMR in cases with a poor biopsy core quality sample precluding any histological diagnosis, ii) underscore the antibody-mediated nature of the rejection process found in cases with histological diagnosis not meeting the full AMR criteria according to the Banff score classification (Loupy et al, Am J Transplant. 2020 May 28. doi: 10.1111/ajt. 15898), iii) highlight the preponderance of the antibody-mediated effector process in cases with Banff mixed rejections and most importantly, iv) guide the type of rescue therapy decision-making according to the effector mechanisms driving rejection.

A second aspect of the invention thus provides an *in vitro* method for recommending a medical regime for preventing and/or treating allograft rejection in a patient, the method comprising: (a) diagnosing if the patient suffers from allograft rejection by the method as defined above and (b) recommending a medical regimen for preventing and/or treating allograft rejection if the patient is diagnosed of suffering from allograft rejection. Also disclosed herein is a method of treating allograft rejection, which method comprises (a) diagnosing if the patient suffers from allograft rejection by the method as defined above and (b) administering a medical regimen for treating allograft rejection if the patient is diagnosed of suffering from allograft rejection. Since the present method differentially detects AMR, the medical regimen may be advantageously optimized for treating this particular type of rejection.

The inventors have further found that determining the level of expression of *SIGLEC17P* and/or at least one of its associated downstream coding genes may also be useful for determining the response to a medical regime that is administered to the patient to treat allograft rejection.

Thus, a third aspect of the invention refers to a method to stablish the response of a patient suffering from allograft rejection to a medical regime for the treatment of allograft rejection, which method comprises determining the level of expression of *SIGLEC17P* and/or at least one of its associated downstream coding genes in an isolated sample from the patient being treated and comparing said level of expression with that determined for the same patient before the treatment or at an earlier phase of the treatment, wherein a reduction of the level of expression with respect to before the treatment or earlier phase of the treatment is indicative of a good response to the medical regime.

A fourth aspect of the invention refers to use of a kit for the diagnosis of allograft rejection particularly, AMR, the kit comprising means for determining the level of expression of *SIGLEC17P* and/or of at least one of its associated downstream coding genes selected from the group consisting of *AP4S1, ZMYM6, USP21, DMAP1, SUPT5H, TP53BP1, NECAB3, BTD, DACT1,* and combinations thereof, together with instructions for comparing the results with reference values.

Finally, a fifth aspect of the invention refers to the use of *SIGLEC17P* and/or at least one of its associated downstream coding genes selected from the group consisting of *AP4S1, ZMYM6, USP21, DMAP1, SUPT5H, TP53BP1, NECAB3, BTD, DACT1,* and combinations thereof, as biomarkers for the diagnosis of allograft rejection, for recommending a medical regimen for treating allograft rejection or for stablishing the response of a patient suffering from allograft rejection to a medical regimen for the treatment of allograft rejection.

**Brief Description of Drawings**

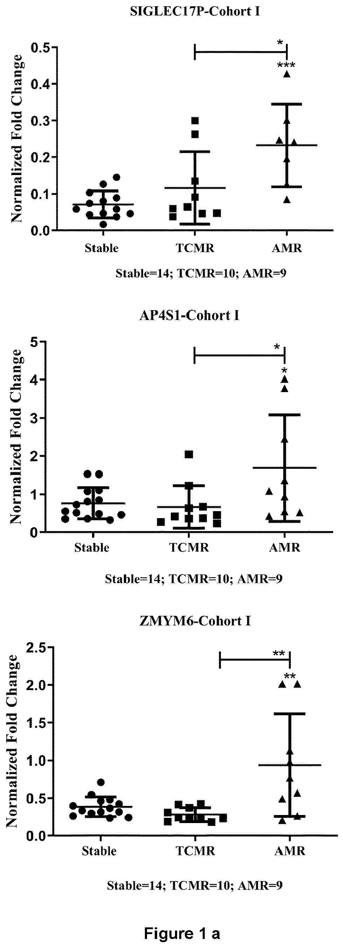

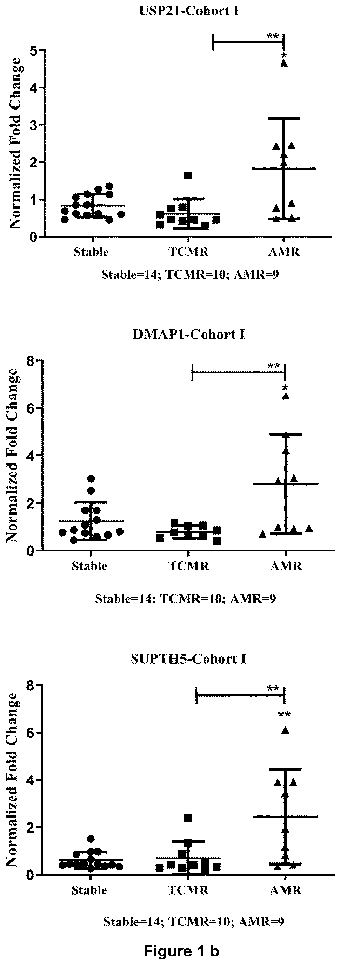

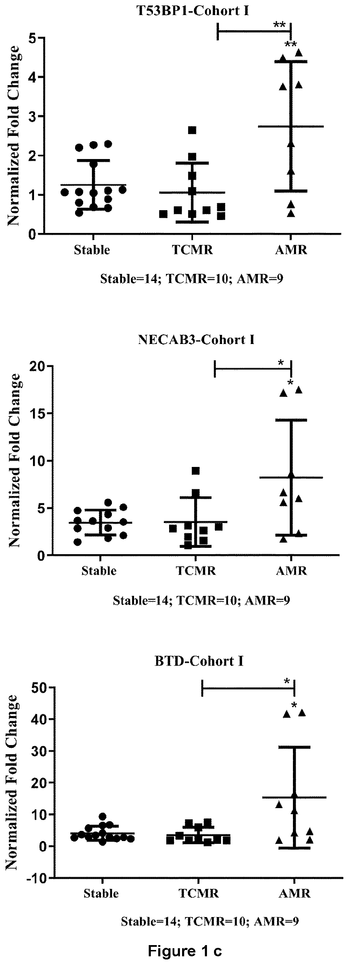

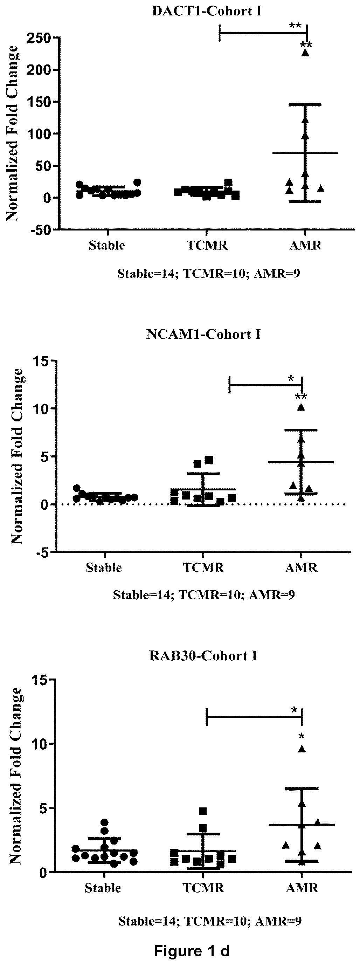

**Figure. 1.** Bar graphs showing normalized mRNA expression of *SIGLEC17P* and its associated coding genes. One-way ANOVA was used to determine significant differences between groups and Tuckey multiple comparison test to compare the difference between each pair of means. **a-d.** Differential gene expression in validation cohort I. *SIGLEC17P*-AMR vs. TCMR: p=0.022, AMR vs. STA: p= 0.0007, STA: 0.07125±0.03705, AMR: 0.2323± 0.1133, TCMR: 0.1160±0.09869; *AP4S1*- AMR vs. TCMR: p=0.031, AMR vs. STA: p=0.037, STA: 0.7652 ± 0.4108, AMR: 1.687 ± 1.399, TCMR: 0.6691 ± 0.5628; *ZMYM6*- AMR vs. TCMR: p= 0.0014, AMR vs. STA: p= 0.0038, STA: 0.3861± 0.1316, AMR: 0.9380± 0.6814, TCMR: 0.2818± 0.09229; *USP21*- AMR vs. TCMR: p= 0.0043, AMR vs.

STA: p= 0.0120, STA: 0.8361 ± 0.3075, AMR: 1.832± 1.350, TCMR: 0.6185± 0.3997; *DMAP1-* AMR vs. TCMR: p= 0.0049, AMR vs. STA: p= 0.0195, STA: 1.251± 0.7931, AMR: 2.806± 2.091, TCMR: 0.7854± 0.2629; *SUPT5H-* AMR vs. TCMR: p= 0.0056, AMR vs. STA: p= 0.0017, STA: 0.6141± 0.3482, AMR: 2.453± 1.996, TCMR: 0.7128± 0.6880; *TP53BP1-* AMR vs. TCMR: p= 0.0037, AMR vs. STA: p= 0.0061, STA:1.254± 0.6196, AMR: 2.743± 1.650, TCMR: 1.056± 0.7488; *NECAB3-* AMR vs. TCMR: p= 0.0305, AMR vs. STA: p= 0.0188, STA: 3.483± 1.314, AMR: 8.225± 6.061, TCMR: 3.541± 2.566; *BTD-* AMR vs. TCMR: p= 0.0131, AMR vs. STA: p= 0.0108, STA: 4.098± 2.208, AMR: 15.35± 15.89, TCMR: 3.554± 2.415; *DACT1-* AMR vs. TCMR: p= 0.0071, AMR vs. STA: p= 0.0046, STA: 10.04± 6.806, AMR: 69.81± 75.63, TCMR: 9.828± 6.197; NCAM1- AMR vs. TCMR: p= 0.0154, AMR vs. STA: p= 0.0013, STA: 0.7785± 0.3715, AMR: 4.438± 3.340, TCMR: 1.540± 1.667; RAB30- AMR vs. TCMR: p=0.0422, AMR vs. STA: p=0.0342, STA:1.707±0.916, AMR: 3.68±2.823, TCMR: 1.644±1.351.

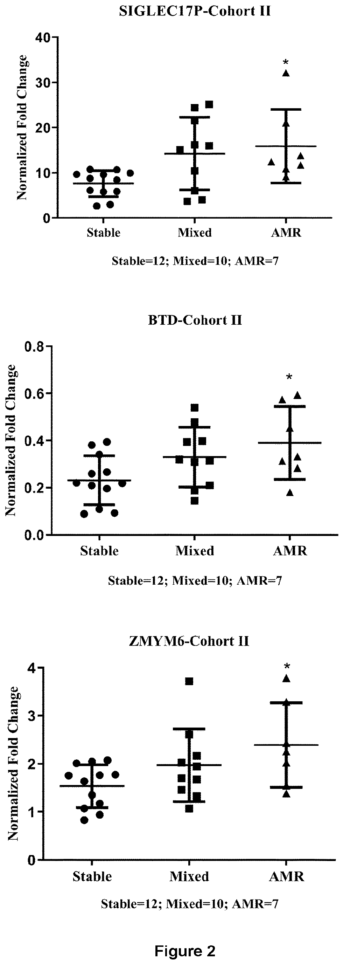

**Figure. 2.** Bar graphs showing normalized mRNA expression of *SIGLEC17P* and is associated coding genes. One-way ANOVA was used to determine significant differences between groups and Tuckey multiple comparison test to compare the difference between each pair of means. Differential gene expression in validation cohort II. *SIGLEC17P-* AMR vs. STA: p= 0.0296, STA: 7.618± 2.855, AMR: 15.91± 8.117; *BTO* - AMR vs. STA: p= 0.0334, STA: 0.2316±0.1042, AMR: 0.3905±0.1548; *ZMYM6* - AMR vs. STA: p= 0.0416, STA: 1.537±0.4455, AMR: 2.391 ± 0.8787.

## Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "transplantation" and variations thereof refers to the insertion of a transplant (also called graft) into a recipient, whether the transplantation is syngeneic (where the donor and recipient are genetically identical), allogeneic (where the donor and recipient are of different genetic origins but of the same species), or xenogeneic (where the donor and recipient are from different species).

The term "allograft" or "allogenic transplant" or "allotransplant" is the transplanted tissue, for example, an organ, from a donor that is of different genetic origins but of the same species as the recipient. For example, the allograft may be a solid organ, in particular, a kidney, but also a lung, heart, pancreas, liver, etc. The allograft may be any other kind of tissue, such as skin, bone, muscle, vascular tissue, cartilage, etc.

As used herein, the term "allotransplant rejection" or "allograft rejection" encompasses both acute and chronic transplant rejection and refers to rejection of the transplanted tissue by the immune system of the recipient.

"Antibody-mediated rejection" (herein for short "AMR") is, as defined by the revised Banff classification (Loupy *et* al, supra), a condition in which histologic evidence of injury is associated with evidence of current/recent antibody interaction with vascular endothelium and serologic evidence of donor-specific antibodies (DSA) to human leukocyte antigen (HLA) or non-HLA antigens ABMR manifests as microcirculation lesions and transcript changes reflecting endothelial injury, interferon-$\gamma$ effects, and natural killer cells.

"T cell-mediated rejection" (herein for short "TCMR") is characterized by infiltration of the interstitium by T cells and macrophages, intense IFNG and TGFB effects, and epithelial deterioration.

The term "diagnosis" is known to the person skilled in the art. As used herein "diagnosis" is understood as becoming aware of a particular medical condition, complication; the determination of the nature of the condition; or the distinguishing of the condition from another. It refers both to the process of attempting to determine or identify the possible condition, and to the opinion reached by this process. A diagnosis, in the sense of diagnostic procedure, can be regarded as an attempt at classification of an individual's condition into separate and distinct categories that allow medical decisions about treatment and prognosis to be made.

The term "reference value" in the context of the present invention is to be understood as a predefined level of the biomarker in a sample or group of samples. The samples are taken from a subject or group of subjects wherein the presence, absence, stage, or course of the disease has been properly performed previously. This value is used as a threshold to discriminate subjects wherein the condition to be analysed is present from those wherein such condition is absent. The skilled person in the art, making use of the general knowledge, is able to choose the subject or group of subjects more adequate for obtaining the reference value. Methods for obtaining the reference value from the group of subjects selected are well known in the state of the art (Burtis C. A. et al., 2008, Chapter 14, section "Statistical Treatment of Reference Values"). As will be apparent to the skilled person the reference values will be defined according to the purpose, target population for the diagnosis, balance between specificity and sensibility, etc.

The term "biomarker", as used herein, refers generally to a molecule, i.e., a gene (or pseudogene) or protein, the expression of which in a biological sample from a patient can be detected by standard methods in the art (as well as those disclosed herein), and is predictive or denotes a condition of the patient from which it was obtained.

The present application refers to a method for diagnosing allograft rejection in a patient which can also advantageously identify if the rejection is antibody-mediated.

Previous transcriptional studies performed to obtain a better understanding of the underlying molecular mechanisms that lead to rejection have been centered on coding genes. Despite the advances in understanding of the overall biology of transplant rejection provided by said studies, the distinct differences in molecular pathways regulating TCMR and AMR have been difficult to dissect in any biologically meaningful manner.

The present inventors have gone beyond the previous approaches and studied the changes in microRNAs or long non-coding RNAs, which are not usually measured by standard microarray technologies. As a result, the inventors have found that the non-functional Sialic Acid Binding Ig Like Lectin 17 Pseudogene *(SIGLEC17P)* is significantly upregulated in patients that suffer from antibody-mediated allograft rejection but not in those suffering from T cell-mediated rejection. Additionally, the inventors have found that at least 9 *SIGLEC17P* direct downstream associated coding genes are also significantly upregulated in patients suffering from AMR. In particular, the associated coding genes are *AP4S1, ZMYM6, USP21, DMAP1, SUPT5H, TP53BP1, NECAB3, BTD* and *DACT1.* The association between these genes and *SIGLEC17P* is that *AP4S1, ZMYM6, USP21, DMAP1, SUPT5H, TP53BP1, NECAB3, BTD* and *DACT1* are direct downstream coding genes from *SIGLEC17P.*

The human Sialic Acid Binding Ig Like Lectin 17 Pseudogene (*SIGLEC17P*, NCBI Reference Sequence NC_000019.10: 51167328-51173524, Gene ID: 284367) is a non-functional gene which is nevertheless still expressed at high levels in some cells.

*AP4S1* gene (NCBI Reference Sequence: NC_000014.9: 31025106-3109645, Gene ID: 11154) is the gene encoding adaptor related protein complex 4 subunit sigma 1 in humans.

*ZMYM6* gene (NCBI Reference Sequence NC_000001.11:c35031945-34986165; Gene ID: 9204) is the gene encoding Zinc finger MYM-type protein 6 in humans.

*USP21* gene (NCBI Reference Sequence NC_000001.11:161159487-161165752; Gene ID: 27005) is the gene encoding ubiquitin carboxyl-terminal hydrolase 21 in humans.

*DMAP1* gene (NCBI Reference Sequence NC_000001.11:44213471-44220673; Gene ID: 55929) is the gene encoding DNA methyltransferase 1-associated protein 1 in humans.

*SUPT5H* gene (NCBI Reference Sequence NC_000019.10:39445546-39476670; Gene ID: 6829) is the gene encoding transcription elongation factor SPT5 in humans.

*TP53BP1* gene (NCBI Reference Sequence NC_000015.10:c43510640-43403061; Gene ID: 7158) is the gene encoding tumor suppressor p53-binding protein 1 in humans.

*NECAB3* gene (NCBI Reference Sequence NC_000020.11:c33674428-33657087; Gene ID: 63941) is the gene encoding N-terminal EF-hand calcium-binding protein 3 in humans.

*BTD* gene (NCBI Reference Sequence NC_000003.12:15601352-15722516; Gene ID: 686) is the gene encoding Biotinidase in humans.

*DACT1* gene (NCBI Reference Sequence NC_000014.9:58634061-58648321; Gene ID: 51339) is the gene encoding disheveled binding antagonist of beta catenin 1 in humans.

The inventors have further found that the *SIGLEC17P* pseudogene and nine of its associated downstream coding genes comprise a genetic signature involved in the physiophatological mechanisms that lead to AMR and are useful as biomarkers for the differential diagnosis of AMR.

Accordingly, the first aspect of the invention provides a method for diagnosing allograft rejection that comprises determining the level of expression of *SIGLEC17P* and/or of at least one of its associated downstream coding genes in an isolated sample from the patient. In an particular embodiment the method of the invention is for diagnosing antibody-mediated allograft rejection. Advantageously, the method of the invention effectively detects patients undergoing an AMR. This may mean patients that only suffer from AMR or patients suffering from both underlying mechanisms, AMR and TCMR, but not patients solely having TCMR. Thus, in a particular embodiment of the first aspect of the invention the method is for the differential diagnosis of antibody mediated rejection.

The differential diagnosis of AMR allows to stratify the patients in an AMC group and a non-AMC group. A particular embodiment thus provides a method for stratifying a patient into AMC group and non-AMC group, the method comprising the level of expression of *SIGLEC17P,* wherein when the level of expression of *SIGLEC17P* is higher than a reference value, this correlates with the AMC group. The non-AMR group may comprise patients that do not suffer from allograft rejection or patients who suffer from TCMR. The AMC group may comprise patients who suffer AMR alone or AMR and TCMR.

*SIGLEC17P* may be used in the method of the invention on its own or in combination with one or more of its associated coding genes. In a particular embodiment, the method of the invention comprises determining the level of expression of *SIGLEC17P.* In other embodiments, the method comprises determining, in addition to *SIGLEC17P,* the level of expression of at least one of its associated downstream coding genes. In particular embodiments the at least one associated downstream coding gene is selected from the group consisting of *AP4S1, ZMYM6, USP21, DMAP1,*

*SUPT5H, TP53BP1, NECAB3, BTD, DACT1* and combinations thereof. The method of the invention also contemplates determining the level of expression of a *SIGLEC17P*-associated coding gene, preferably selected from *AP4S1, ZMYM6, USP21, DMAP1, SUPT5H, TP53BP1, NECAB3, BTD, DACT1* and combinations thereof, on its/their own.

In other embodiments *SIGLEC17P* and/or at least an associated coding gene may be used in combination with other biomarkers of allograft rejection diagnosis. For example, as shown in the examples below, the NCAM/CD56 transcript, as well as the B-cell related protein coding gene *TP53BP1,* were also found to be significantly over expressed in AMR as compared to TCMR and STA. Thus, NCAM/CD56 and/or *TP53BP1* are also useful as biomarkers for diagnosing allograft rejection, in particular AMR, and may be employed in the method of the invention, alone or in combination with *SIGLEC17P* and/or its associated downstream coding genes. Preferably NCAM/CD56 and/or *TP53BP1* are used in combination with *SIGLEC17P.*

NCAM (NCBI Reference Sequence NC_000011.10:112961420-113278436; Gene ID: 4684), also called CD56, is the gene encoding homophilic binding glycoprotein in humans.

*TP53BP1* (NCBI Reference Sequence NC_000015.10:c43510640-43403061; Gene ID: 7158) is the gene encoding tumor suppressor p53-binding protein 1 in humans.

Determining the level of expression in the method of the invention includes qualitative and/or quantitative determination (i.e. detecting and/or measuring the expression level). The expression level as determined may be a relative expression level or an absolute level of expression. The level of expression may be determined by determining the quantity of mRNA or the quantity of translated protein. In the case of non-coding genes, such as *SIGLEC17P* pseudogene, the expression level is determined as the mRNA. As for the coding genes, their level of expression may be determined as the mRNA or as the corresponding translated protein.

In order to determine the mRNA, the first step is usually extracting the nucleic acid contained in the biological samples (e.g., peripheral blood obtained from the patient), for example, using lytic enzymes or chemical solutions or extracted by nucleic-acid-binding resins following the manufacturer's instructions. The extracted mRNA is then detected by hybridization (e.g., Northern blot analysis) and/or amplification (e.g., reverse transcription Polymerase Chain Reaction "RT-PCT"). Quantitative or semi-quantitative RT-PCR is often employed. In a preferred embodiment of the first aspect of the invention the level of expression is determined by quantitative reverse transcription PCR (qRT-PCR). Real-time quantitative or semi-quantitative RT-PCR is particularly advantageous. Primers designed to align with the target sequences are designed to allow for the amplifications. Thus, in some embodiments, determining the level of expression comprises using primers for amplifying the target sequences. In particular embodiments the amplification is performed by qRT-PCR. The amplification requires further reagents, such as nucleotides, enzymes, buffers, etc. Quantitative PCR additionally requires that the nucleotides are labelled. Particular embodiments thus contemplate using labelled nucleotides or labelled primers or labelled probes for determining the level of expression in the method of the invention.

In the case of the coding genes, the quantity of the corresponding proteins may also be determined by methods well known to the skilled person, such as immunochemistry. The term "immunochemistry" as used herein refers to a variety of techniques for detecting antigens (in the present case any of the proteins encoded by the above genes or antigenic fragments thereof) in a sample by exploiting the principle of antibodies binding specifically to the target protein(s). Visualizing an antibody-antigen interaction can be then accomplished in a number of ways, usually by conjugating the antibody to an enzyme, such as peroxidase, that can catalyse a colour-producing reaction, or to a label (for example a fluorophore, such as fluorescein or rhodamine). The immunochemistry technique can be direct or indirect.

The term "antibody or a fragment thereof able to bind to the target protein(s)" is to be understood as any immunoglobulin or fragment thereof able to selectively bind the target protein(s) referred in the aspects and embodiments of the present invention. It includes monoclonal and polyclonal antibodies. The term "fragment thereof" encompasses any part of an antibody having the size and conformation suitable to bind an epitope of the target protein. Suitable fragments include F(ab), F(ab') and Fv. An "epitope" is the part of the antigen being recognized by the immune system (B-cells, T-cells or antibodies).

The means for determining the level of expression of the target genes as described above (primers, probes, antibodies or fragments thereof, aptamers, nucleotides, reagents for qRT-PCR or immunochemistry, etc) form part of a kit.

The level of expression, once determined, is then compared with a reference value. The reference value for performing the method of the invention is the level of expression for each gene determined in a sample or group of samples obtained from a patient or group of patients that have not developed allograft rejection to the same type of tissue (stable transplant) or from a patient or group of patients who do show rejection to the same allograft tissue but whose rejection is not caused by AMR, for example patients undergoing TCMR. Preferably, the reference value is obtained from stable transplant patients. Preferably, the reference value is the level of expression for each gene determined in the blood, for example, peripheral blood, of a stable transplant patient or, more preferably, a group of stable transplant patients.

The skilled person may use any available method to establish the comparison between the level of expression of the target genes in the sample obtained from the patient and the reference value. For instance, when qPCR is used for determining the level of expression, the comparison may be established by using the comparative Ct method. The "Ct" or "Ct value" of a qPCT reaction for a given target nucleic acid has the sense generally given in the art, i.e., the cycle threshold value. The Ct value means the number of PCR cycles where the reporter dye signal is sufficiently high to cross an automatically or manually determined threshold value, and it is a relative measure of the concentration of nucleic acid target in the qPCR reaction.

The comparative Ct method is also known as deltadeltaCt (2-ΔΔCT) method, where:

$$\Delta\Delta Ct = \Delta Ct(patient) - \Delta Ct(control\ subjects),$$

and

$$\Delta Ct = Ct(transcript\ of\ interest) - Ct(transcript\ of\ the\ housekeeping\ gene)$$

The housekeeping gene may be selected according to parameters well known to the skilled person.

When the level of expression of *SIGLEC17P* and/or of at least one of its associated downstream coding genes is higher than the reference value, this is indicative of allograft rejection. In the sense of the present invention, the expression "higher than a reference value" is understood as any increase in the level of expression.

The sample in which the level of expression of the genes is determined may be any biological sample obtained from the patient. The sample may be for example a biopsy of the allograft tissue. In particular embodiments the sample is a biological fluid such as blood, plasma, serum, saliva, urine, exudate, cerebrospinal fluid, sputum, etc. Preferably, the sample is selected from blood, plasma or serum, more preferably, peripheral blood.

A second aspect of the invention is related to recommending a medical regime for preventing and/or treating allograft rejection in a patient by performing the method of diagnosis of the first aspect of the invention, and recommending a medical regime for preventing and/or treating allograft rejection when it is determined that the patient suffers therefrom. All embodiments described above for the first aspect of the invention also apply for the second aspect.

Since the method of the invention provides additional information, namely, it provides a diagnosis of antibody-mediated rejection, in particular embodiments the medical regime that is recommended is specific for treating antibody-mediated rejection. This is clinically relevant in order to provide the patients with the treatment option that is most appropriate for their condition. It is also relevant for optimizing healthcare resources. Moreover, the inventors found that *SIGLEC17P* pseudogene and its associated downstream coding genes are possibly involved in the physiophatological mechanisms that lead to AMR. As such, the method of the invention may detect the rejection on a very early stage, before significant damage is done to the transplanted tissue, and the process may be rectified by administration of an appropriate medical regime. Allograft survival may be subsequently greatly enhanced thanks to the method of the invention.

In particular embodiments the medical regime comprises administering B cell-depleting antibodies, complement inhibitors, proteasome inhibitors, cyclophosphamide, interleukin-6 inhibitors, plasmapheresis, splenectomy or combinations thereof. In particular embodiments, the medical regime comprises administering gammaglobulin, rituximab, bortezomib, eculizumab, plasmapheresis or combinations thereof.

The patients have variable responses to the administered medical regimes. Sometimes the first treatment option provides no improvement or results in intolerable side effects. It is useful to monitor the response of the patient to the applied medical regime in order to maintain, modify or complement the on-going treatment. The present invention provides, in its third aspect, a method to stablish the response of a patient suffering from allograft rejection to a medical regime for its treatment. This aspect involves determining the level of expression of *SIGLEC17P* and/or at least one of its associated downstream coding genes in an isolated sample from the patient being treated and comparing said level of expression with that determined for the same patient before the treatment or at an earlier phase of the treatment. If the level of expression is reduced this means that the treatment is working. If the level of expression is increased, then this may be indicative of the medical regime not being effective, or at least not sufficiently effective. In such case the medical practitioner may recommend interrupting the treatment and/or administering a different medical regime. The medical practitioner may also recommend a change of dosis, administration regime or complement therapy.

Again, all embodiments described above for the first and second aspects of the invention regarding the biomarkers, determination methods, reagents, samples obtained from the patient, medical regimes, etc, also apply for this third aspect.

Also disclosed is kit for performing methods of the first, second or third aspects of the invention as defined above. Said kit contains means for determining the level of expression of *SIGLEC17P* and/or at least one of its associated downstream coding genes, often together with instructions enabling the comparison of the results of said determination with reference values in order to make the prognosis.

In some embodiments the means for determining the level of expression are means for determining mRNA.

Said means may include primers for amplification, probes, nucleotides, reagents and buffers for amplification, hybridization and/or extraction or of the mRNA from the biological sample. In particular embodiments the means comprise primers for amplifying the target genes (SIGELC17P and/or associated coding genes) and optionally other reagents for the amplification, for example by qRT-PCR. In other embodiments the kit comprises means for determining the proteins encoded by the target genes. Said means may include antibodies, fragments thereof, aptamers or any such binding molecules that specifically bind one or more of the *SIGLEC17P*-associated coding genes. In particular embodiments said means are for performing ELISA or lateral flow immunochromatography. In some embodiments, the kit comprises one or more solid surfaces for determining the level of expression of the biomarkers. In one embodiment, the solid surface comprises a microarray chip. In another embodiment, the solid surface comprises a bead. In another embodiment, the solid surface comprises a lateral flow stripe. In a further embodiment, the solid surface comprises a nanoparticle. In one embodiment, the kit comprises means for determining the level of expression of the gene(s) in a biological sample from an individual who has received a renal allograft.

In some embodiments the package or kit further comprises a reference standard. Said reference standard may be a reference sample or group of samples, or the predetermined reference values. In particular embodiment the reference standard is a cut-off value. The package or kit may also comprise internal references, such as housekeeping genes. In some embodiments the package or kit comprises instructions to determine the level of expression and for comparing the results with the reference in order to provide a diagnosis according to the invention.

The present invention further provides for the use of the kits as defined above for the diagnosis of allograft rejection. Particular embodiments provide the use of said kits for the diagnosis of AMR.

The method of the invention may be automated in order to provide a diagnosis result. Thus, the invention also provides a system for determining the diagnosis of allograft rejection, in particular, of antibody-mediated rejection, in a patient comprising data processing means, said data processing means been configured:

- to assess in a test sample obtained from the patient the level of expression of *SIGLEC17P* and/or at least one of its associated downstream coding genes;
- to determine whether said levels of *SIGLEC17P* and/or at least one of its associated downstream coding genes are above predetermined reference values; and
- to determine the diagnosis of allograft rejection by evaluating the result of the previous determination.

The in vitro methods of the invention generally provide for determining the diagnosis of allograft rejection, in particular, of antibody-mediated rejection, in a patient and for recommending an appropriate medical regime for the treatment of said patient. In some embodiments, said methods may further comprise the steps of (i) collecting the diagnostic information, and (ii) saving the information in a data carrier.

In the sense of the invention a "data carrier" is to be understood as any means that contain meaningful information data for determining the diagnosis of allograft rejection, in particular, of antibody-mediated rejection, in a patient and/or recommending an appropriate medical regime, such as paper. The carrier may also be any entity or device capable of carrying the prognosis data or information for recommending an appropriate therapy. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the diagnosis data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

The present invention also discloses the following numbered embodiments:

1. An *in vitro* method for diagnosing allograft rejection in a patient, the method comprising determining in an isolated sample from the patient the level of expression of Sialic Acid Binding Ig Like Lectin 17 Pseudogene *(SIGLEC17P)* and/or of at least one of its associated downstream coding genes, wherein when the level of expression of *SIGLEC17P* and/or of at least one of its associated downstream coding genes is higher than a reference value, this is indicative of allograft rejection.

2. The *in vitro* method according to embodiment 1, wherein the allograft rejection is antibody-mediated rejection.

3. The *in vitro* method according to any of the embodiments 1-2, that is for the differential diagnosis of antibody mediated rejection and T cell-mediated rejection.

4. The *in vitro* method according to any of the preceding embodiments, wherein the at least one *SIGLEC17P*-associated downstream coding gene is selected from the list consisting of *AP4S1, ZMYM6, USP21, DMAP1, SUPT5H, TP53BP1, NECAB3, BTD, DACT1* and combinations thereof.

5. The *in vitro* method according to any of the preceding embodiments, wherein the sample is a body fluid.

6. The *in vitro* method according to the preceding embodiment, wherein the body fluid is selected from blood, plasma and serum.

7. The *in vitro* method according to any of the preceding embodiments, the method comprising determining the level of expression of *SIGLEC17P.*

8. The in vitro method according to any of the preceding embodiments, the method comprising determining the level of expression of *SIGLEC17P* and at least one of the *SIGLEC17P*-associated downstream coding genes.

9. The *in vitro* method according to any of the preceding embodiments, the method comprising determining the level of at least one of the *SIGLEC17P*-associated downstream coding genes.

10. The *in vitro* method according to any of the preceding embodiments, further comprising determining the level of expression of at least one additional gene selected from NCAM1/CD56 and *TP53BP1.*

11. The *in vitro* method according to any of the preceding embodiments, wherein the reference value is the level of expression of each gene determined in a biological sample obtained from a patient or group of patients that have not developed allograft rejection to the same type of tissue.

12. The *in vitro* method according to any of the preceding embodiments, wherein the sample is a body fluid.

13. The *in vitro* method according to the preceding embodiment, wherein the body fluid is selected from blood, plasma or serum.

14. The *in vitro* method according to the preceding embodiment, wherein the body fluid is peripheral blood.

15. The *in vitro* method according to any of the preceding embodiments, wherein determining the level of expression comprises determining mRNA.

16. The *in vitro* method according to the preceding embodiment, wherein the mRNA is determined by quantitative reverse transcription PCR.

17. The *in vitro* method according to any of the embodiments 15-16, wherein the mRNA is determined by using primers for amplification of *SIGLEC17P* and/or at least one of the *SIGLEC17P*-associated downstream coding genes.

18. The *in vitro* method according to the preceding embodiment, wherein said primers form part of a kit.

19. The *in vitro* method according to any of the embodiments 1-14, wherein determining the level of expression of a *SIGLEC17P*-associated downstream coding gene comprises determining the amount of the encoded protein.

20. The *in vitro* method according to the preceding embodiment, wherein the amount of protein is determined by immunochemistry.

21. The *in vitro* method according to any of the embodiments 19-20, wherein the protein is determined by using antibodies or fragments thereof.

22. The *in vitro* method according to the preceding embodiment, wherein said antibodies of fragments thereof form part of a kit.

23. The *in vitro* method according to any of the preceding embodiments, wherein the allograft is selected from kidney, lung, heart, pancreas and liver.

24. The *in vitro* method according to the preceding embodiment, wherein the allograft is a kidney.

25. A method for stratifying a patient suffering from allograft rejection into antibody-mediated rejection group and non-antibody-mediated rejection group, the method comprising diagnosing if the patient suffers from antibody-mediated rejection by the method as defined in any of the preceding embodiments and placing the patient in the antibody-mediated rejection group when the level of expression of *SIGLEC17P* and/or at least one of its associated downstream coding genes is higher than a reference value.

26. An *in vitro* method for recommending a medical regime for preventing and/or treating allograft rejection in a patient, the method comprising

a) diagnosing if the patient suffers from allograft rejection by the method as defined in any of the embodiments 1-24 and
b) recommending a medical regime for preventing and/or treating allograft rejection if the patient is diagnosed of suffering from allograft rejection.

27. The *in vitro* method according to the preceding embodiment, wherein the medical regime is specific for treating antibody-mediated rejection.

28. The *in vitro* method according to the preceding embodiment, wherein the medical regime comprises administering immunoglobulin, B cell-depleting antibodies, complement inhibitors, proteasome inhibitors, cyclophosphamide, interleukin-6 inhibitors, plasmapheresis, splenectomy or combinations thereof.

29. The *in vitro* method according to the preceding embodiment, wherein the medical regime comprises administering gammaglobulin, rituximab, bortezomib, eculizumab, plasmapheresis or combinations thereof.

30. A method to stablish the response of a patient suffering from allograft rejection to a medical regime for the treatment of allograft rejection, which method comprises determining the level of expression of *SIGLEC17P* and/or at least one of its associated downstream coding genes in an isolated sample from the patient being treated and comparing said level of expression with that determined for the same patient before the treatment or at an earlier phase of the treatment, wherein an reduction of the level of expression with respect to before the treatment or earlier phase of the treatment is indicative of a good response to the medical regime.

31. The *in vitro* method according to embodiment 30, wherein the allograft rejection is caused by antibody-mediated rejection.

32. The *in vitro* method according to any of the embodiments 30-31, wherein the at least one *SIGLEC17P*-associated downstream coding gene is selected from the list consisting of *AP4S1, ZMYM6, USP21, DMAP1, SUPT5H, TP53BP1, NECAB3, BTD, DACT1* and combinations thereof.

33. The *in vitro* method according to any of the embodiments 30-32, wherein the sample is a body fluid.

34. The *in vitro* method according to the preceding embodiment, wherein the body fluid is selected from blood, plasma and serum.

35. The *in vitro* method according to the preceding embodiment, wherein the body fluid is peripheral blood.

36. The *in vitro* method according to any of the embodiments 30-35, wherein determining the level of expression comprises determining mRNA.

37. The *in vitro* method according to the preceding embodiment, wherein the mRNA is determined by quantitative reverse transcription PCR.

38. The *in vitro* method according to any of the embodiments 36-37, wherein the mRNA is determined by using primers for amplification of the target gene.

39. The *in vitro* method according to the preceding embodiment, wherein said primers form part of a kit.

40. The *in vitro* method according to any of the embodiments 30-35, wherein determining the level of expression of the target gene comprises determining the amount of the encoded protein.

41. The *in vitro* method according to the preceding embodiment, wherein the amount of protein is determined by immunochemistry.

42. The *in vitro* method according to any of the embodiments 40-41, wherein the protein is determined by using antibodies or fragments thereof.

43. The *in vitro* method according to the preceding embodiment, wherein said antibodies of fragments thereof form part of a kit.

44. The *in vitro* method according to any of the embodiments 30-43, wherein the allograft is selected from kidney, lung, heart, pancreas and liver.

45. The *in vitro* method according to the preceding embodiment, wherein the allograft is a kidney.

46. Use of a kit for the diagnosis of allograft rejection, the kit comprising means for determining the level of expression of *SIGLEC17P* and/or of at least one of its associated downstream coding genes and instructions for comparing the results with reference values.

47. The use according to any of the preceding embodiment, wherein the at least one *SIGLEC17P*-associated downstream coding gene is selected from the list consisting of *AP4S1, ZMYM6, USP21, DMAP1, SUPT5H, TP53BP1, NECAB3, BTD, DACT1* and combinations thereof.

48. The use according to any of the claims 46-47, wherein the allograft rejection is antibody-mediated rejection.

49. The use according to any of the embodiments 46-48, wherein the means are for determining mRNA.

50. The use according to the preceding embodiment, wherein the means for determining mRNA comprise primers for amplification of the target gene.

51. The use according to any of the embodiments 46-50, wherein the means for determining *SIGLEC17P.*

52. The use according to any of the embodiments 46-48, wherein the means are for determining encoded protein.

53. The use according to the previous embodiment, wherein the means comprise antibodies or fragments thereof.

54. The use according to any of the embodiments 46-53, further comprising a solid support.

55. The use according to any of the embodiments 46-54, further comprising a reference sample or predetermined reference values.

55. Use of *SIGLEC17P* and/or at least one of its associated downstream coding genes as biomarkers for the diagnosis of allograft rejection, for recommending a medical regime for treating allograft rejection or for stablishing the response of a patient suffering from allograft rejection to a medical regime for the treatment of allograft rejection.

56. The use according to embodiment 14, that is for the diagnosis of antibody mediated rejection.

57. The use according to any of the embodiments 55-56, wherein the at least one *SIGLEC17P*-associated downstream coding gene is selected from the list consisting of *AP4S1, ZMYM6, USP21, DMAP1, SUPT5H, TP53BP1, NECAB3, BTD, DACT1* and combinations thereof.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

**Examples**

**Methods**

Patients of the Study

A total of 100 kidney transplant patients were evaluated in this study. A first cohort of 37 consecutive, adult kidney transplant recipients undergoing either for-cause or surveillance biopsies between January 2014 and December 2015 at Bellvitge University Hospital (Barcelona, Spain) were enrolled in the study. This cohort was named the discovery cohort. Three different groups of patients were categorized according to the histological diagnosis made by a blinded expert transplant pathologist following the updated Banff 2017 classification 26; patients with pure acute TCMR (n=13); patients with antibody-mediated rejection (AMR, n=12) and patients with a normal/preserved kidney allograft parenchyma (STA , n=12). All blood samples were obtained at the time of the kidney allograft biopsy and before any immunosuppression rescue therapy was given. Subsequently, 63 additional kidney transplant patients from two distinct transplant centers undergoing consecutives either for-cause or surveillance biopsies between January 2017 and October 2018 were evaluated for targeted mRNA expression of most representative non-coding and coding-related genes observed in the first discovery cohort of patients; Out of these validation patients, 33 were from Bellvitge University Hospital, Barcelona, Spain (cohort I) and 29 from University California San Francisco, San Francisco, USA (cohort II). In cohort I, main histological diagnoses in for-cause biopsies were: TCMR (n=10) and AMR (n=9), whereas 8 patients were evaluated in surveillance biopsies and classified as STA (n=15). In the cohort II, main histological diagnoses were: STA (n=12), AMR (n=7) and MIXED rejections (n=10). Each biopsy was scored by a site's specialist pathologist blinded for any result of the transcriptional study. All patients of the study were receiving the same triple immunosuppressive-based therapy with tacrolimus, mycofenolate mofetil and prednisone. All patients evaluated in the study gave written informed consent to participate and the institutional review board at Bellvitge University Hospital and UCSF approved the study (Num PR228/13, Num 14-13573).

Renal allograft histology

Kidney allograft biopsies were performed for cause due to kidney allograft dysfunction in all patients with TCMR and AMR rejection whereas STA patients showed stable allograft function and a preserved graft parenchyma in 6-month surveillance biopsies. All renal biopsies were analyzed following the Banff 2017 score classification (Haas et al, American Journal of Transplantation 2018; 18: 293-307) and the histological analysis was blindly evaluated by an expert renal pathologist at each center prior to submitting samples for molecular evaluation.

Gene expression analysis

Blood samples were collected in PAXgene Blood RNA Tubes. The tubes contain a reagent that lyses blood cells and immediately stabilizes intracellular RNA to preserve the gene expression profile. Blood samples collected in PAXgene Blood RNA Tubes can be safely stored or transported at 15-25°C. RNA was purified from the stabilized blood samples with the PAXgene Blood miRNA Kit following manufacturer's instructions. The RNA obtained included both mRNA and small RNAs such as miRNA.
A total of 50-ng RNA was reversed transcribed into complementary DNA (cDNA) using SuperScript VILO (Invitrogen, Thermo Fischer Scientific, Foster City, CA) and then amplified in a target specific amplification step for *SIGLEC17P* and other associated genes using TaqMan PreAmpMaster Mix and TaqMan Primers and Probes (Invitrogen, Thermo Fischer Scientific, Foster City, CA) for a total of 18 amplification cycles. QPCR reactions were performed in the Fluidigm BioMark FD system using 18S gene as a housekeeping gene and Human XpressRef Universal Total RNA (Qiagen, Valencia, CA) as a reference RNA for a total of 40 cycles. Resulting chip data was initially analyzed for quality control using the BioMark Analysis Software Version 2.0 (Fluidigm, South San Francisco, CA) and Ct values were exported into Excel. Normalization of the data was done in two steps. Ct values of individual genes were normalized against Ct value of 18S for each gene to get dCt values. dCt values of each sample was normalized against dCt values of the reference sample to get ddCt values which was subsequently used to calculate fold change (RQ) values for each gene in each sample.

**Results**

Clinical and histological characteristics of the discovery cohort

Main baseline clinical characteristics of the discovery patient cohort is depicted in Table 1.

**Table 1. Main clinical and demographic characteristics of discovery cohort.**

| Main clinical variables | Clinical Phenotypes | | | |
|---|---|---|---|---|
| | TCMR (n=13) | AMR (n=12) | STA (n=12) | p value |
| Donor Age (years) | 61.5±14.09 | 43.2±21.92 | 50.6±17.24 | 0.048 |
| Recipient Age (years) | 62.2±12.25 | 45.7±15.26 | 55.3±11.98 | 0.013 |
| Recipient Sex (F) | 5 (38.5%) | 4 (33.3%) | 5 (41.7%) | 0.91 |
| Donor Sex (F) | 8 (61.5%) | 5 (41.7%) | 5 (41.7%) | 0.51 |
| Cause of ESRD (%)<br>Unknown<br>Glomerular<br>Interstitial<br>Vascular<br>Diabetes<br>APKD<br>Others | <br>6(46)<br>4(31)<br>0(0)<br>1(7.7)<br>2(15.4)<br>0(0)<br>0(0) | <br>3(25)<br>5(42)<br>2(17)<br>1(8.3)<br>0(0)<br>0(0)<br>1 (8.3) | <br>5(36)<br>2(16.7)<br>1(8.3)<br>2(16.7)<br>1(8.3)<br>1(8.3)<br>0(0) | 0.55 |
| Type of transplant (Deceased) | 10(77) | 11(91.7) | 11(91.7) | 0.46 |
| Number of transplants<br>1 vs > 1 | 1.15±0.38<br>11 (85%) | 1.6±0.79<br>7 (58%) | 1.17±0.39<br>10 (83%) | 0.1<br>0.23 |
| Number HLA antigen Mismatch | 3.0±0.95 | 3.58±0.9 | 3.25±1.3 | 0.41 |
| Induction type:<br>None<br>anti-CD25 mAb<br>rATG | <br>0 (0%)<br>10 (76.9%)<br>3(23.1%) | <br>2 (16.7%)<br>4 (33.3%)<br>6 (50.0%) | <br>1 (8.3%)<br>10 (83.3%)<br>1 (8.3%) | <br>0.064 |
| DSA at biopsy (yes)<br>Class I | 0 | 12 (100%)<br>3 | 0 | <0.001 |
| ClassII<br>Class I&II | | 7<br>2 | | |
| eGFR at biopsy (ml/min) | 30.1±20.42 | 28.8±20.9 | 49.7±14.42 | 0.016 |
| Proteinuria at biopsy (gr/24h) | 0.76±0.9 | 1.79±1.37 | 0.19±0.23 | 0.001 |
| Graft loss after biopsy assessment | 4 (30.8%) | 8 (66.7%) | 1 (8.3%) | 0.01 |
| Time to biopsy (months) | 4.8±3.8 | 91.6±83.1 | 6.8±2.4 | <0.001 |

Abbreviations: ESRD: end stage renal disease; APKD: Autosomic Polycystic disease, HLA: human leukocyte antigens; mAb: monoclonal antibodies; rATG. Rabbit anti-thymocyte globulin; ; DSA: donor-specific antibodies; eGFR: estimated glomerular filtration rate.

TCMR and AMR patients showed worse allograft function than STA patients who showed a good and stable kidney allograft function. Only AMR patients displayed DSA. TCMR Banff 30 scores ranged between IA and IIA (6 IA, 6 IB and 3 IIA).

In table 2 all semi-quantitative histology scores defining the distinct clinico-pathological groups of the study are described.

**Table 2. Main histological lesions of the patients of the discovery cohort.**

| Mean Banff scores in all kidney graft compartments | Histological Phenotypes | | | |
|---|---|---|---|---|
| | TCMR (n=13) | AMR (n=12) | STA (n=12) | P value |
| **Acute lesions:** | | | | |
| ag | 0.8±0.8 | 1.5±0.9 | 0.2±0.4 | <0.001 |
| ai | 2.1±0.8 | 1.1±0.7 | 0.2±0.6 | <0.001 |
| at | 2.3±0.6 | 0.8±0.5 | 0.5±0.9 | <0.001 |
| ti | 2.1±0.9 | 1.4±0.8 | 0.2±0.4 | <0.001 |
| ptc | 0.5±1.0 | 1.0±0.8 | 0.09±0.3 | 0.030 |
| av | 0.2±0.8 | 0.2±0.4 | 0.08±0.3 | 0.81 |
| C4d | 0.1±0.4 | 1.7±1.0 | 0.1±0.6 | <0.001 |
| **Chronic lesions:** | | | | |
| cg | 0.08±0.3 | 1.7±1.3 | 0.08±0.3 | <0.001 |
| ci | 0.8±0.4 | 1.5±0.8 | 0.8±0.8 | 0.03 |
| ct | 0.7±0.5 | 1.7±0.7 | 0.8±0.8 | 0.001 |
| cv | 0.3±0.5 | 0.7±0.9 | 0.2±0.6 | 0.15 |
| ah | 0.1±0.5 | 1.1±1.2 | 0.2±0.6 | 0.01 |
| cm | 0±0 | 0.9±1.1 | 0.08±0.3 | 0.002 |
| Ag: acute glomeruli; ai: acute interstitium; at: acute tubuli; ti: total interstitial inflammation; ptc: peritubular capillaritis; av:acute vascular; cg: chronic glomeruli; ci: chronic interstitium;; ct: chronic tubuli; cv: chronic vascular; ah: arterial hyalinosis; Cm: Chronic mesangial.<br>TCMR: at, ai, av, cv<br>AMR: ag, ptc, c4d, cg, cv+ DSA/antiHLAb<br>IFTA (interstitial fibrosis tubular atrophy): ci, ct | | | | |

Characterization of non-coding genes and related coding genes in two validation cohorts of kidney transplant patients

To understand the fundamental molecular mechanisms underlying each histological phenotype, RNA sequencing (RNAseq) was used to profile unique peripheral blood samples from the discovery cohort. This study identified unique protein-coding and non-coding genes that are specifically associated with antibody-mediated rejection (results not shown). Next, the level of expression of selected non-coding genes and related coding genes were validated in peripheral blood using qRT-PCR in two additional cohorts of kidney transplants showing distinct types of allograft rejections, TCMR, AMR and Mixed rejection as well as stable. Main clinical, immunological and histological characteristics of the two validation cohorts (cohort I and cohort II) are shown in Table 3.

**Table 3. Main demographic, clinical and Immunological variables of the two validation cohorts**

| Variables | Study Phenotypes Validation Cohort I (BCN) | | | |
|---|---|---|---|---|
| | TCMR (n=10) | AMR (n=9) | STABLE (n=14) | P value |
| **Time After Transplant (months)** | 27.64±29.13 | 57.22±27.10 | 6.37±0.77 | 0.001 |
| **Donor Age (years)** | 30.00±7.90 | 48.75±14.70 | 51.70±11.77 | 0.012 |
| **Recipient Age (years)** | 38.00±15.30 | 48.50±14.01 | 52.80±12.62 | 0.042 |
| **Recipient Gender (F)** | 3 (30) | 8 (66.7) | 3 (20) | 0.038 |
| **Donor Gender (F)** | 4 (40) | 4 (44.4) | 10 (66.7) | 0.356 |
| **Cause of ESRD (%)** | | | | 0.274 |
| Unknown | 4 (40) | 2 (16.7) | 4 (26.7) | |
| Glomerular | 0 (0) | 1 (8.3) | 1 (6.7) | |
| Interstitial | 2 (20) | 0 (0) | 0 (0) | |
| Vascular | 0 (0) | 2 (16.7) | 2 (13.3) | |
| Diabetes | 3 (30) | 1 (8.3) | 3 (20) | |
| APKD | 0 (0) | 4 (33.3) | 2 (13.3) | |
| Others | 1 (10) | 2 (16.7) | 3 (20) | |
| **Number HLA Mismatch** | 4.40±1.07 | 4.33±1.15 | 3.86±1.68 | 0.563 |
| **Induction type:** | | | | 0.346 |
| No induction | 1 (10) | 0 (0) | 1 (6.7) | |
| Basiliximab | 6 (60) | 4 (33.3) | 9 (60) | |
| rATG | 3 (30) | 8 (66.7) | 5 (33.3) | |

| DSA (yes) | 0 (0) | 9 (100) | 0 (0) | <0.001 |
|---|---|---|---|---|
| Class I | 0 (0) | 4 (44.4) | 0 (0) | |
| Class II | 0 (0) | 4 (44.4) | 0 (0) | |
| Class I&II | 0 (0) | 1 (11.1) | 0 (0) | |
| **eGFR time BX (ml/min)** | 25.44±15.62 | 39.58±19.02 | 56.00±11.39 | <0.001 |
| **Graft loss (yes)** | 4 (40) | 0 (0) | 0 (0) | 0.002 |
| **Mean Banff scores** <br> **Acute lesions:** | | | | |
| acute glomeruli (ag) | 0.40±0.69 | 1.90±0.99 | 0.00±0.00 | <0.001 |
| acute interstitium (ai) | 2.30±1.06 | 1.58±0.99 | 0.13±0.35 | <0.001 |
| acute tubuli (at) | 2.40±0.97 | 0.75±1.13 | 0.06±0.25 | <0.001 |
| peritubular capillaritis (ptc) | 0.50±0.85 | 1.58±1.08 | 0.00±0.00 | <0.001 |
| acute vascular (av) | 0.40±0.69 | 0.27±0.65 | 0.00±0.00 | 0.147 |
| C4d | 0.30±0.06 | 0.91±1.31 | 0.00±0.00 | 0.007 |
| **Chronic lesions:** | | | | |
| chronic glomeruli (cg) | 0.00±0.00 | 0.50±0.85 | 0.00±0.00 | 0.021 |
| chronic interstitium (ci) | 0.60±0.69 | 0.60±0.85 | 0.00±0.00 | 0.018 |
| chronic tubuli (ct) | 0.50±0.85 | 0.50±0.85 | 0.13±0.35 | 0.250 |
| chronic vascular (cv) | 0.00±0.00 | 0.30±0.67 | 0.06±0.25 | 0.214 |

| Variables | Study Phenotypes <br> Validation Cohort II (UCSF) | | | |
|---|---|---|---|---|
| | **MIXED** <br> **(n=10)** | **AMR** <br> **(n=7)** | **STABLE** <br> **(n=12)** | **P value** |
| **Time After Transplant (months)** | 43.3±53.38 | 91.86±70.94 | 42.66±13.89 | 0.0761 |
| **Donor Age (years)** | 38.10±15.31 | 37.29±16.04 | 31.17±16.08 | 0.5292 |
| **Recipient Age (years)** | 44.1±14.38 | 43.63±5.85 | 42±12.66 | 0.9188 |
| **Recipient Gender (F)** | 5 (50) | 2 (28) | 4 (33) | 0.6977 |
| **Donor Gender (F)** | 3 (30) | 4 (57) | 8 (66) | 0.2338 |
| **Cause of ESRD (%)** | | | | 0.8573 |
| Unknown | 0 (0) | 1 (14.2) | 2 (16.6) | |
| Glomerular | 2 (20) | 1 (14.2) | 2 (16.6) | |
| Interstitial | 3 (30) | 1 (14.2) | 3 (25) | |
| Vascular | 2 (20) | 0 (0) | 3 (25) | |
| Diabetes | 1 (10) | 2 (28.58) | 1 (8.3) | |
| APKD | 1 (10) | 2 (28.58) | 1 (8.3) | |
| Others | 1 (10) | 0 (0) | 0 (0) | |
| **Number HLA Mismatch** | 4.29±1.60 | 4.72±0.98 | 4.72±1.3 | 0.7544 |
| **Induction type:** | | | | 0.1017 |
| No induction | 3 (30) | 4 (57.14) | 0 (0) | |
| Basiliximab | 3 (30) | 2 (28.57) | 6 (50) | |
| rATG | 4 (40) | 1 (14.29) | 6 (50) | |
| **DSA (yes)** | | | | 0.0169 |
| Class I | 7 (70) | 5 (71.4) | 2 (16.6) | |

| | | | | |
|---|---|---|---|---|
| Class II | 0 (0) | 4 (57.14) | 0 (0) | |
| Class I&II | 0 (0) | 4 (57.14) | 0 (0) | |
| | 0 (0) | 4 (57.14) | 0 (0) | |
| eGFR time BX (ml/min) | 46±26.20 | 37.38±17.86 | 78.25±18.87 | <0.001 |
| Graft loss (yes) | 0 (0) | 0 (0) | 0 (0) | 1 |
| **Mean Banff scores** <br> **Acute lesions:** | | | | |
| acute glomeruli (ag) | 0.9±0.99 | 2.86±0.338 | 0.00±0.00 | <0.001 |
| acute interstitium (ai) | 1.9±0.99 | 0.5±0.53 | 0.00±0.00 | <0.001 |
| acute tubuli (at) | 2.2±1.14 | 0.5±0.53 | 0.00±0.00 | <0.001 |
| peritubular capillaritis (ptc) | 1.2±1.09 | 1.3±0.95 | 0.00±0.00 | <0.001 |
| acute vascular (av) | 0.44±0.73 | 0.00±0.00 | 0.00±0.00 | 0.0432 |
| C4d | 0.2±0.42 | 1.3±1.4 | 0.00±0.00 | <0.001 |
| **Chronic lesions:** | | | | |
| chronic glomeruli (cg) | 0.11±0.33 | 1.6±1.4 | 0.00±0.00 | <0.001 |
| chronic interstitium (ci) | 1.2±0.79 | 1.1±0.93 | 0.00±0.00 | <0.001 |
| chronic tubuli (ct) | 1.3±0.67 | 1.1±0.93 | 0.00±0.00 | <0.001 |
| chronic vascular (cv) | 1.6±1.3 | 1.4±1.3 | 0.00±0.00 | <0.001 |

The selected genes included the pseudogene Sialic acid-recognizing Ig-like lectins 17P *(SIGLEC17P)* and 9 *SIGLEC17P*-associated coding genes, the Natural Killer (NK)-related transcript (NCAM1/CD56) due to its association with *SIGLEC17P* and a highly up-regulated coding gene associated with B-cell immunoglobulin class switch recombination (*TP53BP1*). The non-coding SIGLEC 17P gene was of particular interest because, even though it is a non-coding gene, it is still highly expressed in high levels in NK cells, which is widely accepted to mediate AMR. SIGLEC 13 and 17 were rendered non-functional during Hominin evolution. While the SIGLEC 17P underwent a single base pair deletion in humans, it is still preserved in chimpanzees and other new world monkeys. The SIGLEC 17P's mRNA is still widely expressed in NK cells. This non-coding gene was significantly enriched in the common GO term and is also found to be up-regulated in AMR (downregulated in TCMR) by the RNAseq study.

The validation study confirmed the significant association of this non-coding gene with AMR. In cohort I (Figure 1a-d), expression of *SIGLEC17P* was also significantly upregulated in AMR patients as compared with TCMR and STA. Notably, the nine coding genes associated with *SIGLEC17P (AP4S1, ZMYM6, USP21, DMAP1, SUPT5H, TP53BP1, NECAB3, BTD, DACT1)* were also found to be highly upregulated in AMR as compared to TCMR and STA. Likewise to *SIGLEC17P,* the matured NK cell specific marker NCAM1/CD56 as well as the B-cell related protein coding gene *TP53BP1,* were also found to be significantly over expressed in AMR as compared to TCMR and STA. Likewise, in cohort II (Figure 2) the expression of *SIGLEC17P* was found to be upregulated in AMR than STA but not than mixed rejections.

In conclusion, a significantly increased expression of *SIGLEC17P* and 9 different direct downstream coding genes together with the NCAM/CD56 transcript was observed in AMR as compared to stable and TCMR patients. Moreover, the selectivity of this gene was further observed when patients with mixed rejection were also included in the validation analysis. As shown, this gene expression did also capture the humoral pattern of these mixed phenotypes whereas it did not in pure TCMR patients thus, suggesting the potential value of evaluating these genes for identifying the type of allograft rejection and guide decision-making regarding the type of rescue therapy.

**Citation List**

Roedder S, et al. "The kSORT Assay to Detect Renal Transplant Patients at High Risk for Acute Rejection: Results of the Multicenter AART Study", PLoS Med. 2014 Nov 11;11(11):e1001759.

Friedewald, J. et al. (2018). "Development and Clinical validity of a novel blood-based molecular biomarker for sub-clinical acute rejection following kidney transplant. American Journal of Transplantation". doi:10.1111/ajt.15011

Loupy et al. "The Banff 2019 Kidney Meeting Report (I): Updates on and clarification of criteria for T cell- and antibody-mediated rejection", Am J Transplant. 2020 May 28. doi: 10.1111/ajt.15898.

Burtis C. A. et al., 2008, Chapter 14, section "Statistical Treatment of Reference Values".

**Claims**

1. An *in vitro* method for diagnosing allograft rejection in a patient, the method comprising determining in an isolated sample from the patient the level of expression of Sialic Acid Binding Ig Like Lectin 17 Pseudogene *(SIGLEC17P)* and/or of at least one of its associated downstream coding genes selected from the group consisting of adaptor related protein complex 4 subunit sigma 1 gene *(AP4S1)*, Zinc finger MYM-type protein 6 gene *(ZMYM6)*, ubiquitin carboxyl-terminal hydrolase 21 gene *(USP21),* DNA methyltransferase 1-associated protein 1 gene *(DMAP1)*, transcription elongation factor SPT5 gene *(SUPT5H)*, tumor suppressor p53-binding protein 1 gene *(TP53BP1)*, N-terminal EF-hand calcium-binding protein 3 gene *(NECAB3)*, Biotinidase gene *(BTD)*, disheveled binding antagonist of beta catenin 1 gene *(DACT1)*, and combinations thereof, wherein when the level of expression of *SIGLEC17P* and/or of at least one of its associated downstream coding genes is higher than a reference value, this is indicative of allograft rejection.

2. The *in vitro* method according to claim 1, wherein the allograft rejection is antibody-mediated rejection.

3. The *in vitro* method according to any of the preceding claims, the method comprising determining the level of expression of *SIGLEC17P.*

4. The *in vitro* method according to any of the preceding claims, wherein the sample is selected from blood, plasma and serum.

5. The *in vitro* method according to any of the preceding claims, wherein the allograft is selected from kidney, lung, heart, pancreas and liver allografts, preferably, a kidney allograft.

6. The *in vitro* method according to any of the preceding claims, wherein determining the level of expression comprises determining mRNA.

7. The *in vitro* method according to the preceding claim, wherein the mRNA is determined by using primers for amplification of the target genes by quantitative Reverse Transcription Polymerase Chain Reaction.

8. The *in vitro* method according to the preceding claim, wherein said primers form part of a kit.

9. An *in vitro* method for recommending a medical regime for treating allograft rejection in a patient, the method comprising

   a) diagnosing if the patient suffers from allograft rejection by the method as defined in any of the claims 1-8 and
   b) recommending a medical regime for preventing and/or treating allograft rejection if the patient is diagnosed of suffering from allograft rejection.

10. The *in vitro* method according to the preceding claim, wherein the medical regime is specific for treating antibody-mediated rejection.

11. The *in vitro* method according to the preceding claim, wherein the medical regime is selected from immunoglobulins, B cell-depleting antibodies, complement inhibitors, proteasome inhibitors, cyclophosphamide, interleukin-6 inhibitors, plasmapheresis or splenectomy, preferably administering gammaglobulin, rituximab, bortezomib, eculizumab or plasmapheresis.

12. A method to stablish the response of a patient suffering from allograft rejection to a medical regime for the treatment of allograft rejection, which method comprises determining the level of expression of *SIGLEC17P* and/or at least one of its associated downstream coding genes selected from the group consisting of *AP4S1, ZMYM6, USP21,*

*DMAP1, SUPT5H, TP53BP1, NECAB3, BTD, DACT1,* and combinations thereof, in an isolated sample from the patient being treated and comparing said level of expression with that determined for the same patient before the treatment or at an earlier phase of the treatment, wherein a reduction of the level of expression with respect to before the treatment or an earlier phase of the treatment is indicative of a good response to the medical regime.

13. Use of a kit for the diagnosis of allograft antibody-mediated rejection, the kit comprising means for determining the level of expression of *SIGLEC17P* and/or of at least one of its associated downstream coding genes selected from the group consisting of *AP4S1, ZMYM6, USP21, DMAP1, SUPT5H, TP53BP1, NECAB3, BTD, DACT1,* and combinations thereof, and instructions for comparing the results with reference values.

14. Use of *SIGLEC17P* and/or at least one of its associated downstream coding genes selected from the group consisting of *AP4S1, ZMYM6, USP21, DMAP1, SUPT5H, TP53BP1, NECAB3, BTD, DACT1,* and combinations thereof, as biomarkers for the diagnosis of allograft rejection, for recommending a medical regime for treating allograft rejection or for stablishing the response of a patient suffering from allograft rejection to a medical regime for the treatment of allograft rejection.

15. The use according to claim 14, that is for the differential diagnosis of antibody mediated rejection and T cell-mediated rejection.

SIGLEC17P-Cohort I

Stable=14; TCMR=10; AMR=9

AP4S1-Cohort I

Stable=14; TCMR=10; AMR=9

ZMYM6-Cohort I

Stable=14; TCMR=10; AMR=9

**Figure 1 a**

## USP21-Cohort I

Stable=14; TCMR=10; AMR=9

## DMAP1-Cohort I

Stable=14; TCMR=10; AMR=9

## SUPTH5-Cohort I

Stable=14; TCMR=10; AMR=9

Figure 1 b

## T53BP1-Cohort I

Stable=14; TCMR=10; AMR=9

## NECAB3-Cohort I

Stable=14; TCMR=10; AMR=9

## BTD-Cohort I

Stable=14; TCMR=10; AMR=9

Figure 1 c

**DACT1-Cohort I**

Stable=14; TCMR=10; AMR=9

**NCAM1-Cohort I**

Stable=14; TCMR=10; AMR=9

**RAB30-Cohort I**

Stable=14; TCMR=10; AMR=9

Figure 1 d

SIGLEC17P-Cohort II

Stable=12; Mixed=10; AMR=7

BTD-Cohort II

Stable=12; Mixed=10; AMR=7

ZMYM6-Cohort II

Stable=12; Mixed=10; AMR=7

Figure 2

Europäisches
Patentamt

European
Patent Office

Office européen
des brevets

**EUROPEAN SEARCH REPORT**

Application Number

EP 20 38 2620

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/100259 A1 (ICAHN SCHOOL MED MOUNT SINAI [US]) 15 June 2017 (2017-06-15) * the whole document * | 1-15 | INV. C12Q1/6883 G01N33/50 |
| X | WO 2010/142751 A1 (TC LAND EXPRESSION [FR]; INST NAT SANTE RECH MED [FR] ET AL.) 16 December 2010 (2010-12-16) * the whole document * | 1-15 | |
| X | S. M. KURIAN ET AL: "Molecular Classifiers for Acute Kidney Transplant Rejection in Peripheral Blood by Whole Genome Gene Expression Profiling", AMERICAN JOURNAL OF TRANSPLANTATION, vol. 14, no. 5, 11 May 2014 (2014-05-11), pages 1164-1172, XP055172952, ISSN: 1600-6135, DOI: 10.1111/ajt.12671 * the whole document * | 1-15 | |
| X | L. LI ET AL.: "A Peripheral Blood Diagnostic Test for Acute Rejection in Renal Transplantation", AMERICAN JOURNAL OF TRANSPLANTATION, vol. 12, no. 10, 25 September 2012 (2012-09-25), pages 2710-2718, XP055322481, DK ISSN: 1600-6135, DOI: 10.1111/j.1600-6143.2012.04253.x * the whole document * | 1-15 | TECHNICAL FIELDS SEARCHED (IPC) C12Q G01N |
| X | US 10 443 100 B2 (SCRIPPS RESEARCH INST [US]; UNIV NORTHWESTERN [US]) 15 October 2019 (2019-10-15) * column 1, line 39 - column 5, line 15 * * examples 1-2; table 4 * | 1-15 | |

-/--

The present search report has been drawn up for all claims

| Place of search | Date of completion of the search | Examiner |
|---|---|---|
| Munich | 27 November 2020 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | SILVIA PINEDA ET AL: "Peripheral Blood RNA Sequencing Unravels a Differential Signature of Coding and Noncoding Genes by Types of Kidney Allograft Rejection", KIDNEY INTERNATIONAL REPORTS, vol. 5, no. 10, 26 July 2020 (2020-07-26), pages 1706-1721, XP055753878, ISSN: 2468-0249, DOI: 10.1016/j.ekir.2020.07.023 * the whole document * ----- | | |

TECHNICAL FIELDS
SEARCHED      (IPC)

The present search report has been drawn up for all claims

| Place of search | Date of completion of the search | Examiner |
|---|---|---|
| Munich | 27 November 2020 | Bradbrook, Derek |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 3 936 625 A1**

## ANNEX TO THE EUROPEAN SEARCH REPORT
## ON EUROPEAN PATENT APPLICATION NO.

EP 20 38 2620

This annex lists the patent family members relating to the patent documents cited in  the above-mentioned European search report.
The members are as contained in the European Patent Office EDP file on
The European Patent Office is in no way liable for these particulars which are merely given for the purpose of information.

27-11-2020

| Patent document cited in search report | | Publication date | Patent family member(s) | | Publication date |
|---|---|---|---|---|---|
| WO 2017100259 | A1 | 15-06-2017 | NONE | | |
| WO 2010142751 | A1 | 16-12-2010 | NONE | | |
| US 10443100 | B2 | 15-10-2019 | US 2017137885 | A1 | 18-05-2017 |
| | | | US 2020208217 | A1 | 02-07-2020 |

EPO FORM P0459

For more details about this annex : see Official Journal of the European Patent Office, No. 12/82

## REFERENCES CITED IN THE DESCRIPTION

**Non-patent literature cited in the description**

- *PLoS Med.,* 11 November 2014, vol. 11 (11), e1001759
- **LOUPY et al.** *Am J Transplant.,* 28 May 2020
- **BURTIS C. A. et al.** Statistical Treatment of Reference Values. 2008
- **HAAS et al.** *American Journal of Transplantation,* 2018, vol. 18, 293-307
- **ROEDDER S et al.** The kSORT Assay to Detect Renal Transplant Patients at High Risk for Acute Rejection: Results of the Multicenter AART Study. *PLoS Med.,* 11 November 2014, vol. 11 (11), e1001759
- **FRIEDEWALD, J. et al.** Development and Clinical validity of a novel blood-based molecular biomarker for sub-clinical acute rejection following kidney transplant. *American Journal of Transplantation,* 2018
- **LOUPY et al.** The Banff 2019 Kidney Meeting Report (I): Updates on and clarification of criteria for T cell- and antibody-mediated rejection. *Am J Transplant.,* 28 May 2020